# EUROPEAN PATENT APPLICATION

(11) **EP 4 234 580 A1**
(43) Date of publication of application: **30.08.2023**
(21) Application number: 21893973.4
(22) Date of filing: 18.11.2021
(51) Int. Cl.: C07K 16/28, C12N 15/13, C07K 16/46, C12N 15/63, C12N 5/10, C07K 19/00, A61K 39/395, A61P 35/00

(54) **NKG2A-TARGETING ANTIBODY AND USE THEREOF**

(30) Priority: 23.11.2020 CN 202011320557
(71) Applicant: Bioheng Therapeutics Limited, Grand Cayman KY1-1209 (KY)
(72) Inventor: ZHOU, Yali, Nanjing, Jiangsu 210061 (CN); CHEN, Gong, Nanjing, Jiangsu 210061 (CN); JIANG, Xiaoyan, Nanjing, Jiangsu 210061 (CN); GUO, Tingting, Nanjing, Jiangsu 210061 (CN); REN, Jiangtao, Nanjing, Jiangsu 210061 (CN); HE, Xiaohong, Nanjing, Jiangsu 210061 (CN); WANG, Yanbin, Nanjing, Jiangsu 210061 (CN); HAN, Lu, Nanjing, Jiangsu 210061 (CN)
(74) Representative: Rimini, Rebecca
(86) International application number: PCT/CN2021/131435
(87) International publication number: WO 2022/105826

(57) **Abstract**

Provided are an NKG2A-targeting antibody, and a multispecific antibody, a chimeric receptor, an antibody conjugate, a pharmaceutical composition and a kit which comprise same, and the use thereof in the diagnosis/treatment/prevention of diseases associated with NKG2A expression.

## Description

### Technical Field

The present disclosure belongs to the field of immunotherapy. More specifically, the present disclosure relates to a NKG2A-targeting antibody and use thereof in the prevention and/or treatment and/or diagnosis of diseases.

### Background Art

Natural killer (NK) cells are a very important type of lymphocytes in the body, which play an important role in both innate immunity and acquired immunity. There are two types of surface receptors on the surface of NK cells, which can be divided into inhibitory receptor and activating receptor according to the their function.They mediate different recognition modes of NK cells and transmit different inhibitory signals and activating signals, respectively. The CD94/NKG2 family is a type of receptor family that has been studied more, mainly including NKG2A, NKG2B, NKG2C, NKG2D, NKG2E, NKG2F, NKG2H and other members. Among them, NKG2A is an inhibitory receptor, and its ligand is the non-classical major histocompatibility complex class I molecule HLA-E. After the HLA-E molecules expressed on the target cells bind with NKG2A, the killing function of NK cells is inhibited. Therefore, antibodies targeting CD94/NKG2A may enhance the killing activity of tumor-specific lymphocytes against tumor cells.

Various anti-NKG2A antibodies have been described in the art. For example, Sivori et al. (Eur J Immunol 1996; 26:2487-92) mention the murine anti-NKG2A antibody Z270; Carretero et al. (EurJ Immunol 1997; 27:563-7) describe the murine anti-NKG2A antibody Z199 (now commercially available from Beckman Coulter, Inc., product number IM2750, USA); Vance et al. (J Exp Med 1999; 190:1801-12) mention the rat anti-mouse NKG2-antibody 20D5 (now commercially available from BD Biosciences Pharmingen, Cat. No. 550518, USA); and US Patent Application 20030095965 describes the murine antibody 3S9, which reportedly binds to NKG2A, NKG2C and NKG2E.

The present disclosure aims to provide a NKG2A-targeting antibody and use thereof in the prevention and/or treatment and/or diagnosis of diseases.

### Summary

In a first aspect, the present disclosure provides a NKG2A-targeting antibody, comprising a light chain variable region and a heavy chain variable region, wherein the light chain variable region comprises CDR-L1 as set forth in SEQ ID NO: 1, CDR-L2 as set forth in SEQ ID NO: 2, and CDR-L3 as set forth in SEQ ID NO: 3, and the heavy chain variable region comprises CDR-H1 as set forth in SEQ ID NO: 4, CDR-H2 as set forth in SEQ ID NO: 5, and CDR-H3 as set forth in SEQ ID NO: 6, wherein the light chain variable region comprises amino acid M at position 21, and amino acid T at position 85, and the heavy chain variable region comprises amino acid M at position 34, amino acid A at position 49, amino acid P at position 61, and amino acid T at position 97.

In an embodiment, the light chain variable region comprises amino acid S or T at position 22, amino acid I or V at position 58, and amino acid L or V at position 104.

In a preferred embodiment, the light chain variable region of the anti-NKG2A antibody has at least 90% identity to an amino acid sequence selected from the group consisting of SEQ ID NOs: 10 and 13, or has one or several (e.g., 2, 3, 4, 5, 6, 7, 8, 9, 10, at most 7, at most 6, at most 5, at most 4, at most 3, at most 2) conservative modifications of amino acids compared to SEQ ID NO: 10 or 13, the heavy chain variable region has at least 90% identity to the amino acid sequence as set forth in SEQ ID NO: 11 or has one or several (e.g., 2, 3, 4, 5, 6, 7, 8, 9, 10, at most 7, at most 6, at most 5, at most 4, at most 3, at most 2) conservative modifications of amino acids compared to the amino acid sequence as set forth in SEQ ID NO: 11. More preferably, the anti-NKG2A antibody comprises a light chain variable region selected from the group consisting of SEQ ID NOs: 10 and 13 and a heavy chain variable region as set forth in SEQ ID NO: 11.

In an embodiment, the anti-NKG2A antibody has an amino acid sequence selected from the group consisting of SEQ ID NOs: 12 and 14.

The present disclosure further provides a nucleic acid molecule encoding the anti-NKG2A antibody as described above. Therefore, in an embodiment, the nucleic acid molecule encoding the anti-NKG2A antibody has at least 90%, at least 91%, at least 92%, at least 93%, at least 94%, at least 95%, at least 96%, at least 97%, at least 98%, at least 99%, or 100% sequence identity to a nucleotide sequence selected from the group consisting of SEQ ID NOs: 16-17, and the anti-NKG2A antibody encoded thereby can specifically bind to NKG2A. Preferably, the nucleic acid molecule encoding the anti-NKG2A antibody is selected form SEQ ID NOs: 16-17.

In another aspect, the present disclosure further provides a multispecific antibody (preferably bispecific antibody or trispecific antibody), which comprises the anti-NKG2A antibody as described above, and one or more second antibodies or antigen-binding portions thereof that specifically bind to antigens different from NKG2A.

In an embodiment, the second antibody or antigen-binding portion thereof may be in the form of any antibody or antibody fragment, such as a full-length antibody, Fab, Fab', (Fab')₂, Fv, scFv, scFv-scFv, a minibody, a diabody or sdAb.

The present disclosure further provides a vector comprising a nucleic acid molecule encoding the anti-NKG2A antibody or the multispecific antibody as described above, and a host cell expressing the anti-NKG2A antibody or the multispecific antibody.

In another aspect, the present disclosure further provides a chimeric receptor comprising one or more NK inhibitory ligands, a transmembrane domain and a signaling domain, wherein the NK inhibitory ligand comprises the anti-NKG2A antibody or the multispecific antibody comprising the anti-NKG2A antibody as described above, wherein the signaling domain comprises one or more costimulatory domains.

In an embodiment, the chimeric receptor comprises two NK inhibitory ligands, wherein the first NK inhibitory ligand is the anti-NKG2A antibody as described above and the second NK inhibitory ligand is selected from: (1) an antibody or a fragment thereof targeting one of the following NK inhibitory receptors: LlR1, NKG2B, CD94, LIR2, LIR3, KIR2DL1, KIR2DL2/3, KIR3DL1, CEACAM1, LAIR1, and KLRG1; or (2) HLA-E, HLA-F, HLA-G, cadherin, collagen, OCIL, sialic acid, PD-L1, PD-L2, CD155, CD112, CD113, Gal-9, FGL1, and NK inhibitory receptor binding regions comprised therein.

In an embodiment, the signaling domain of the chimeric receptor of the disclosure consists of one or more co-stimulatory domains. That is, it does not comprise a primary signaling domain, such as that from FcRγ, FcRβ, CD3γ, CD3δ, CD3ε, CD3ζ, CD22, CD79a, CD79b and CD66d.

In another embodiment, the signaling domain of the chimeric receptor of the present disclosure may further comprise a primary signaling domain, such as the CD3ζ intracellular region.

The present disclosure further provides a nucleic acid molecule encoding the NKG2A-targeting chimeric receptor as defined above, and a vector comprising the nucleic acid molecule.

The present disclosure further provides an engineered immune cell, which expresses the chimeric receptor comprising the anti-NKG2A antibody of the present disclosure, wherein the expression of at least one MHC-related gene is suppressed or silenced.

In an embodiment, the MHC-related gene is selected from the group consisting of: HLA-A, HLA-B, HLA-C, B2M, HLA-DPA, HLA-DQ, HLA-DRA, TAP1, TAP2, LMP2, LMP7, RFX5, RFXAP, RFXANK, CIITA and a combination thereof, and preferably selected from the group consisting of HLA-A, HLA-B, HLA-C, B2M, RFX5, RFXAP, RFXANK, CIITA and a combination thereof.

In an embodiment, the engineered immune cell expressing the chimeric receptor comprising the anti-NKG2A antibody of the present disclosure further comprises suppressed or silenced expression of at least one TCR/CD3 gene, examples of which include TRAC, TRBC, CD3γ, CD3δ, CD3ε, CD3ζ.

In an embodiment, the engineered immune cell provided by the present disclosure further express a chimeric antigen receptor targeting a tumor antigen.

In an embodiment, the immune cell is selected from the group consisting of a T cell, a NK cell, a NKT cell, a macrophage, and a dendritic cell.

In another aspect, the present disclosure further provides an antibody conjugate comprising the anti-NKG2A antibody as defined herein and a second functional structure, wherein the second functional structure is selected from the group consisting of an Fc, a radioisotope, a structure moiety for extending half-life, a detectable marker and a drug.

In an embodiment, the structure moiety for extending half-life is selected from the group consisting of an albumin-binding structure, a transferrin-binding structure, a polyethylene glycol molecule, a recombinant polyethylene glycol molecule, a human serum albumin, a fragment of human serum albumin, and a polypeptide (including an antibody) binding to human serum albumin. In an embodiment, the detectable marker is selected from the group consisting of a fluorophore, a chemiluminescent compound, a bioluminescent compound, an enzyme, an antibiotic resistance gene, and a contrast agent. In an embodiment, the drug is selected from the group consisting of a cytotoxin and an immunomodulator.

In another aspect, the present disclosure further provides a detection kit comprising the antibody, the multispecific antibody, the antibody conjugate or the chimeric receptor described in the present disclosure.

In another aspect, the present disclosure further provides a pharmaceutical composition comprising the antibody, the chimeric receptor, the multispecific antibody, the engineered cell or the antibody conjugate described in the present disclosure, and one or more pharmaceutically acceptable excipients.

In another aspect, the present disclosure further provides a method for treating and/or preventing and/or diagnosing diseases associated with NKG2A expression, comprising administering to a subject the antibody, the chimeric receptor, the multispecific antibody, the antibody conjugate, the engineered immune cell or the pharmaceutical composition as described above.

### Brief Description of Drawings

Figure 1 shows the sequence alignment results of the light chain variable region and the heavy chain variable region of hZ199 and its back mutant antibodies hZ199-V1 and hZ199-V2. The amino acid site of the back mutation is highlighted in gray, and the number shows the position of the mutation site in the light chain variable region and the heavy chain variable region.
Figure 2 shows scFv expression levels of UNKi-T cells containing NKG2A antibodies.
Figure 3 shows the inhibitory effect of UNKi-T cells containing NKG2A antibodies on NK cell killing. Two-way ANOVA was used for analysis, and T test was used for statistical analysis. ** indicates that the P value is less than 0.01, reaching a significant level.

### Detailed Description of Embodiments

Unless otherwise specified, all scientific and technical terms used herein have the same meaning as commonly understood by one of ordinary skill in the art to which this disclosure belongs.

### Anti-NKG2A antibody

In the context of the present disclosure, the "Z199 antibody" is the murine anti-NKG2A antibody Z199 as described by Carretero et al. (Eur J Immunol 1997; 27:563-7), now commercially available from Beckman Coulter, Inc., Product No. IM2750. "hZ199 antibody" refers to a humanized Z199 antibody, which comprises a light chain variable region as set forth in SEQ ID NO: 7, a heavy chain variable region as set forth in SEQ ID NO: 8, with the full-length amino acid sequence as set forth in SEQ ID NO: ID NO: 9.

The new NKG2A antibody provided by the present disclosure is obtained by back mutation based on hZ199, so as to provide an antibody with higher affinity and better effect of inhibiting NK cell killing.

As used herein, the term "antibody" has the broadest meaning understood by those skilled in the art and includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity. The antibodies of the present disclosure may be of any class (e.g., IgG, IgE, IgM, IgD, IgA, etc.) or subclass (e.g., IgG1, IgG2, IgG2a, IgG3, IgG4, IgA1, IgA2, etc.).

Typically, whole antibodies comprise two heavy chains and two light chains disulfide-bonded together, each light chain being disulfide-bonded to a respective heavy chain, to form a "Y" configuration. Each heavy chain comprises a heavy chain variable region (VH) and a heavy chain constant region, wherein the heavy chain variable region comprises three complementarity determining regions (CDRs): CDR-H1, CDR-H2 and CDR-H3, and the heavy chain constant region comprises three constant domains: CH1, CH2 and CH3. Each light chain comprises a light chain variable region (VL) and a light chain constant region, wherein the light chain variable region comprises three CDRs: CDR-L1, CDR-L2 and CDR-L3, and the light chain constant region comprises a constant domain CL. In the heavy/light chain variable regions, the CDRs are separated by more conserved framework regions (FRs). The heavy/light chain variable regions are responsible for the recognition and binding of the antigen, while the constant regions mediates the binding of the antibody to host tissues or factors, including various cells of the immune system (e.g., effector cells) and the first component of the classical complement system.

The precise amino acid sequence boundaries for a given CDR or FR can be readily determined using a number of numbering schemes well known in the art, including: Kabat et al. (1991), "Sequences of Proteins of Immunological Interest," 5th Edition, Public Health Service, National Institutes of Health, Bethesda, MD ("Kabat" numbering scheme); Al-Lazikani et al., (1997) JMB 273, 927-948 ("Chothia" numbering scheme); MacCallum et al., J. Mol. Biol 262:732-745 (1996), "Antibody-antigen interactions: Contact analysis and binding sitetopography," J. Mol. Biol. 262, 732-745" ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," JMol Biol, 2001 Jun 8; 309(3):657-70 ("Aho" numbering scheme); and Martin et al., "Modeling antibody hypervariable loops: a combined algorithm," PNAS, 1989, 86(23):9268-9272 ("AbM" numbering scheme).

The boundaries of a given CDR or FR may vary depending on the scheme used for identification. For example, the Kabat scheme is based on structural alignment, while the Chothia scheme is based on structural information. Both the Kabat and Chothia numbering schemes are based on the sequence length of the most common antibody regions and place certain insertions and deletions ("indels") at different positions, resulting in different numbering. The Contact scheme is based on the analysis of complex crystal structures and is similar in many respects to the Chothia numbering scheme. The AbM scheme is a compromise between the Kabat and Chothia definitions and is based on the scheme used by the AbM antibody modeling software of Oxford Molecular.

Thus, unless otherwise specified, a "CDR" of a given antibody or region thereof (e.g., variable region thereof) is understood to encompass the CDRs defined by any of the above schemes or other known schemes. For example, where it is specified that a particular CDR (e.g., CDR3) contains a given amino acid sequence, it is understood that such a CDR may also have the sequence of the corresponding CDR (e.g., CDR3) as defined by any of the above schemes or other known schemes. Likewise, unless otherwise specified, FRs for a given antibody or region thereof (e.g., variable region thereof) are understood to encompass FRs as defined by any of the above schemes or other known schemes. Amino acid numbering herein follows the Chothia scheme unless otherwise indicated.

As used herein, the term "antibody fragment" or "antigen-binding portion" comprises only a portion of an intact antibody, and typically comprises the antigen-binding site of the intact antibody and thus retains the ability to bind antigen. Examples of antibody fragments of the present disclosure include, but are not limited to: Fab, Fab', F(ab')2, Fd fragment, Fd', Fv fragment, scFv, disulfide-linked Fv (sdFv), antibody heavy chain variable region (VH) or light chain variable region (VL), linear antibody, "diabody" with two antigen binding sites, single domain antibody, nanobody, a natural ligand for the antigen or a functional fragment thereof. Accordingly, an "antibody" of the present disclosure encompasses antibody fragments as defined above.

"Single-chain antibody" and "scFv" are used interchangeably herein and refer to an antibody formed by linking the heavy chain variable region (VH) and the light chain variable region (VL) of an antibody through a linker. The optimal length and/or amino acid composition of the linker can be selected. The length of the linker may significantly affect the folding and interaction of the variable domain of scFv. In fact, if shorter linkers (e.g., with between 5-10 amino acids) are used, intrachain folding may be prevented. For selection of linker size and composition, see, e.g., Hollinger et al., 1993 Proc Natl Acad. Sci. U.S.A. 90:6444-6448; U.S. Patent Application Publication Nos. 2005/0100543, 2005/0175606, 2007/0014794 and PCT Publication Nos. WO2006/020258 and WO2007/024715, the entire contents of which are incorporated herein by reference. A scFv may comprise VH and VL linked in any order, e.g. VH-linker-VL or VL-linker-VH.

In a first aspect, the present disclosure provides a NKG2A-targeting antibody, comprising a light chain variable region and a heavy chain variable region, wherein the light chain variable region comprises CDR-L1 as set forth in SEQ ID NO: 1, CDR-L2 as set forth in SEQ ID NO: 2, and CDR-L3 as set forth in SEQ ID NO: 3, and the heavy chain variable region comprises CDR-H1 as set forth in SEQ ID NO: 4, CDR-H2 as set forth in SEQ ID NO: 5, and CDR-H3 as set forth in SEQ ID NO: 6, wherein the light chain variable region comprises amino acid M at position 21, and amino acid T at position 85, and the heavy chain variable region comprises amino acid M at position 34, amino acid A at position 49, amino acid P at position 61, and amino acid T at position 97.

In an embodiment, the light chain variable region comprises amino acid S or T at position 22, amino acid I or V at position 58, and amino acid L or V at position 104.

In a preferred embodiment, the light chain variable region of the anti-NKG2A antibody has at least 90% identity to an amino acid sequence selected from the group consisting of SEQ ID NOs: 10 and 13, or has one or several (e.g., 2, 3, 4, 5, 6, 7, 8, 9, 10, at most 7, at most 6, at most 5, at most 4, at most 3, at most 2) conservative modifications of amino acids compared to SEQ ID NO: 10 or 13, the heavy chain variable region has at least 90% identity to the amino acid sequence as set forth in SEQ ID NO: 11 or has one or several (e.g., 2, 3, 4, 5, 6, 7, 8, 9, 10, at most 7, at most 6, at most 5, at most 4, at most 3, at most 2) conservative modifications of amino acids compared to SEQ ID NO: 11. More preferably, the anti-NKG2A antibody comprises a light chain variable region selected from the group consisting of SEQ ID NO: 10 and 13 and a heavy chain variable region as set forth in SEQ ID NO: 11.

In an embodiment, the anti-NKG2A antibody has an amino acid sequence selected from the group consisting of SEQ ID NOs: 12 and 14.

As used herein, the term "sequence identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, such as Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

As used herein, the term "conservative modification" refers to an amino acid modification that does not significantly affect or alter the binding characteristics of an antibody or antibody fragment comprising the amino acid sequence. These conservative modifications include amino acid substitutions, additions and deletions. Modifications can be introduced into the antibodies of the present disclosure by standard techniques known in the art, such as site-directed mutagenesis and PCR-mediated mutagenesis. A conservative amino acid substitution is one in which an amino acid residue is replaced by an amino acid residue with a similar side chain. Families of amino acid residues with similar side chains have been defined in the art and include those with basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), non-polar side chain (e.g., alanine, valine, leucine, isoleucine, proline, phenylalanine, methionine, tryptophan), betabranched side chain (e.g., threonine, valine, isoleucine) and aromatic side chain (e.g., tyrosine, phenylalanine, tryptophan, histidine). Conservative modifications can be selected, for example, on the basis of similarity in polarity, charge, solubility, hydrophobicity, hydrophilicity, and/or the amphipathic nature of the residues involved.

In an aspect, the present disclosure further provides a multispecific antibody (preferably a bispecific antibody or a trispecific antibody) comprising the anti-NKG2A antibody as described above, and one or more second antibodies that specifically bind to antigens different from NKG2A.

As used herein, the term "multispecific" means that the antigen binding protein has polyepitopic specificity (i.e., is capable of specifically binding two, three or more different epitopes on one biomolecule or is capable of specifically binding epitopes on two, three or more different biomolecules). As used herein, the term "bispecific" means that an antigen binding protein has two different antigen binding specificities.

In an embodiment, the second antibody may be in the form of any antibody or antibody fragment, such as a full-length antibody, Fab, Fab', (Fab')2, Fv, scFv, scFv-scFv, a minibody, a diabody or sdAb.

### Nucleic acid, vector, host cell

In another aspect, the present disclosure relates to a nucleic acid molecule encoding the anti-NKG2A antibody or multispecific antibody of the present disclosure. The nucleic acid of the present disclosure may be RNA, DNA or cDNA. According to an embodiment of the present disclosure, the nucleic acid of the present disclosure is a substantially isolated nucleic acid.

In an embodiment, the nucleic acid molecule encoding the anti-NKG2A antibody has at least 90%, at least 91%, at least 92%, at least 93%, at least 94%, at least 95%, at least 96%, at least 97%, at least 98%, at least 99%, or 100% sequence identity to a nucleotide sequence selected from the group consisting of SEQ ID NOs: 16-17, and the anti-NKG2A antibody encoded thereby specifically binds NKG2A. Preferably, the nucleic acid molecule encoding the anti-NKG2A antibody is as set forth in SEQ ID NOs: 16-17.

The nucleic acid of the present disclosure may also be in the form of a vector, may be present in a vector and/or may be part of a vector, such as a plasmid, cosmid or YAC. The vector may especially be an expression vector, i.e., a vector providing for expression of the anti-NKG2A antibody in vitro and/or in vivo (i.e., in a suitable host cell, host organism and/or expression system). The expression vector typically comprises at least one nucleic acid molecule of the present disclosure operably linked to one or more suitable expression regulatory elements (e.g., promoter, enhancer, terminator, etc.). Selection of such regulatory elements and their sequences for expression in a particular host is well known to those skilled in the art. Specific examples of regulatory elements and other elements useful or necessary for expression of the anti-NKG2A antibody of the present disclosure include, but are not limited to, promoter, enhancer, terminator, integrator, selectable marker, leader sequence, reporter gene.

In another aspect, the present disclosure further provides a host cell expressing the anti-NKG2A antibody, the multispecific antibody of the present disclosure and/or a host cell containing the nucleic acid or vector of the present disclosure. Preferred host cells of the present disclosure are bacterial cells, fungal cells or mammalian cells.

Suitable bacterial cells include cells of Gram-negative bacterial strains (e.g.,*Escherichia coli* strains, *Proteus* strains, and *Pseudomonas* strains) and Gram-positive bacterial strains (e.g., *Bacillus* strains, *Streptomyces* strains, *Staphylococcus* strains and *Lactococcus* strains).

Suitable fungal cells include cells of species of *Trichoderma, Neurospora,* and *Aspergillus*; or cells of species of *Saccharomyces* (e.g., *Saccharomyces cerevisiae*)*, Schizosaccharomyces* (e.g., *Schizosaccharomyces pombe*)*, Pichia* (e.g., *Pichia pastoris* and *Pichia methanolica*) and *Hansenula.*

Suitable mammalian cells include, for example, HEK293 cells, CHO cells, BHK cells, HeLa cells, COS cells, and the like.

However, amphibian cells, insect cells, plant cells, and any other cells known in the art for expressing heterologous proteins can also be used in the present disclosure.

### Chimeric receptor

In another aspect, the present disclosure further provides a chimeric receptor comprising the anti-NKG2A antibody as described above. Since NKG2A is a NK inhibitory receptor, chimeric receptors comprising the NKG2A antibodies can be used to inhibit the killing effect of NK cells.

In an embodiment, the present disclosure provides a chimeric receptor comprising one or more NK inhibitory ligands, a transmembrane domain and a signaling domain, wherein the NK inhibitory ligand comprises the anti-NKG2A antibody or the multispecific antibody comprising the anti-NKG2A antibody as described above, wherein the signaling domain comprises one or more costimulatory domains.

In an embodiment, the chimeric receptor comprises multiple NK inhibitory ligands, such as two NK inhibitory ligands, wherein the first NK inhibitory ligand is the anti-NKG2A antibody as described above, the second NK inhibitory ligand is an antibody or fragment thereof targeting other NK inhibitory receptors, and/or natural ligands of other NK inhibitory receptors or NK inhibitory receptor binding regions comprised therein.

In an embodiment, the second NK inhibitory ligand is an antibody or fragment thereof targeting a NK inhibitory receptor selected from the group consisting of a NKG2/CD94 component (e.g., NKG2B, CD94); a member of the killer cell Ig-like receptor (KIR) family (e.g., KIR2DL1, KIR2DL2/3, KIR2DL5A, KIR2DL5B, KIR3DL1, KIR3DL2, and KIR3DL3); a member of the leukocyte Ig-like receptor

(LIR) family (e.g., LIR1, LIR2, LIR3, LIR5, and LIR8); a member of the NK cell receptor protein 1 (NKR-P1) family (e.g., NKR-P1B and NKR-P1D); an immune checkpoint receptor (e.g., PD-1, TIGIT, and CD96, TIM3, LAG3); carcinoembryonic antigen-related cell adhesion molecule 1 (CEACAM1); a member of the sialic acid-binding immunoglobulin-like lectin (SIGLEC) family (e.g., SIGLEC7 and SIGLEC9); leukocyte-associated immunoglobulin-like receptor 1 (LAIR1); a member of the Ly49 family (e.g., Ly49A, Ly49C, Ly49F, Ly49G1 and Ly49G4) and killer cell lectin-like receptor G1 (KLRG1). Preferably, the second NK inhibitory ligand is selected from an antibody or fragment thereof targeting a NK inhibitory receptor selected from the group consisting of: NKG2B, CD94, LIR1, LIR2, LIR3, KIR2DL1, KIR2DL2/3, KIR3DL1, CEACAM1, LAIR1 and KLRG1. Still more preferably, the second NK inhibitory ligand is an antibody or fragment thereof targeting a NK inhibitory receptor selected from the group consisting of: CD94, KIR2DL1, KIR2DL2/3, KIR3DL1, CEACAM1, LIR1, LAIR1, and KLRG1.

In an embodiment, the second NK inhibitory ligand is a natural ligand of other NK inhibitory receptors or a NK inhibitory receptor binding region comprised thereof, such as HLA-E, HLA-F, HLA-G, cadherin, collagen, OCIL, sialic acid, an immune checkpoint ligand (e.g., PD-L1/PD-L2, CD155, CD112, CD113, Gal-9, FGL1, etc.), and a NK inhibitory receptor binding region comprised thereof. Preferably, the second NK inhibitory ligand is selected from the group consisting of HLA-E, HLA-F, HLA-G, cadherin, PD-L1, PD-L2, and a NK inhibitory receptor binding region comprised thereof. More preferably, the second NK inhibitory ligand is selected from the group consisting of a HLA-E extracellular region, a HLA-G extracellular region, an E-cadherin extracellular region, a PD-L1 extracellular region and a PD-L2 extracellular region. More preferably, the second NK inhibitory ligand is an E-cadherin extracellular region comprising EC1 and EC2, more preferably comprising EC1, EC2, EC3, EC4 and EC5.

As used herein, the term "transmembrane domain" refers to a polypeptide structure that enables expression of a chimeric receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric receptor binds to the target antigen. The transmembrane domains particularly suitable for use in the present disclosure may be derived from, for example, a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154, and functional fragments thereof. Alternatively, the transmembrane domain may be synthesized and may mainly contain hydrophobic residues such as leucine and valine. Preferably, the transmembrane domain is derived from CD8α chain or CD28, and has at least 70%, preferably at least 80%, more preferably at least 90%, at least 95%, at least 97% or at least 99% or 100% sequence identity to the amino acid sequence as set forth in SEQ ID NO: 17 or 19, or the encoding sequence thereof has at least 70%, preferably at least 80%, more preferably at least 90%, at least 95%, at least 97% or at least 99% or 100% sequence identity to the nucleic acid molecule as set forth in SEQ ID NO: 18 or 20.

As used herein, the term "co-stimulatory domain" refers to an intracellular functional signaling domain from a co-stimulatory molecule, which comprises the entire intracellular portion of the co-stimulatory molecule, or a functional fragment thereof. A "costimulatory molecule" refers to a cognate binding partner that specifically binds to a costimulatory ligand on a T cell, thereby mediating a costimulatory response (e.g., proliferation) of the T cell. Costimulatory molecules include, but are not limited to, MHC class 1 molecules, BTLA, and Toll ligand receptors. Examples of costimulatory domains of the present disclosure include, but are not limited to, intracellular regions derived from the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, LAT, NKG2C, SLP76, PD-1, LIGHT, TRIM, and ZAP70. Preferably, the costimulatory domain of the CAR of the present disclosure is from 4-1BB, CD28 or 4-1BB+CD28. In an embodiment, the 4-1BB co-stimulatory domain has at least 70%, preferably at least 80%, more preferably at least 90%, at least 95%, at least 97% or at least 99% or 100% sequence identity to the amino acid sequence as set forth in SEQ ID NO: 23, or the coding sequence thereof has at least 70%, preferably at least 80%, more preferably at least 90%, at least 95%, at least 97% or at least 99% or 100% sequence identity to the nucleic acid molecule as set forth in SEQ ID NO: 24. In an embodiment, the CD28 co-stimulatory domain has at least 70%, preferably at least 80%, more preferably at least 90%, at least 95%, at least 97% or at least 99% or 100% sequence identity to the amino acid sequence as set forth in SEQ ID NO: 21, or the coding sequence thereof has at least 70%, preferably at least 80%, more preferably at least 90%, at least 95%, at least 97% or at least 99% or 100% sequence identity to the nucleic acid molecule as set forth in SEQ ID NO: 22.

In an embodiment, the signaling domain consists of one or more costimulatory domains, that is, it does not comprise a primary signaling domain, such as a primary signaling domain from FcRγ, FcRβ, CD3γ, CD3δ, CD3ε, CD3ζ, CD22, CD79a, CD79b and CD66d. In another embodiment, the signaling domain of the chimeric receptor of the present disclosure may further comprise a primary signaling domain, such as the CD3ζ intracellular region. In a preferred embodiment, the CD3ζ intracellular region has at least 70%, preferably at least 80%, more preferably at least 90%, at least 95%, at least 97% or at least 99% or 100% sequence identity to the amino acid sequence as set forth in SEQ ID NO: 25 or 27, or the coding sequence thereof has at least 70%, preferably at least 80%, more preferably at least 90%, at least 95%, at least 97% or at least 99% or 100% sequence identity to the nucleic acid molecule as set forth in SEQ ID NO: 26 or 28.

In an embodiment, the chimeric receptor of the present disclosure may further comprise a hinge region located between the antibody and the transmembrane domain. As used herein, the term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region comprises a hinge region portion of CD8α, CD28, an Fcγ RIII α receptor, IgG4, or IgG1, more preferably a hinge from CD8α, CD28 or IgG4, and has at least 70%, preferably at least 80%, more preferably at least 90%, at least 95%, at least 97% or at least 99% or 100% sequence identity to the amino acid sequence as set forth in SEQ ID NO: 33, 35 or 37, or the encoding sequence thereof has at least 70%, preferably at least 80%, more preferably at least 90%, at least 95%, at least 97% or at least 99% or 100% sequence identity to the nucleic acid molecule as set forth in SEQ ID NO: 34, 36 or 38.

In an embodiment, the CAR of the present disclosure may further comprise a signal peptide such that when it is expressed in a cell such as a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. Signal peptides that may be used in the present disclosure are well known to those skilled in the art, for example, signal peptides derived from B2M, CD8α, IgG1, GM-CSFRα, and the like. In an embodiment, the signal peptide that may be used in the present disclosure is from B2M or CD8α, and has at least 70%, preferably at least 80%, more preferably at least 90%, at least 95%, at least 97% or at least 99% or 100% sequence identity to an amino acid sequence as set forth in SEQ ID NO: 29 or 31, or the coding sequence thereof has at least 70%, preferably at least 80%, more preferably at least 90%, at least 95%, at least 97% or at least 99% or 100% sequence identity to the nucleic acid molecule as set forth in SEQ ID NO: 30 or 32.

In an embodiment, the CAR comprises the anti-NKG2A antibody as provided herein or the multispecific antibody comprising the anti-NKG2A antibody, a CD8α transmembrane region and a signaling domain, wherein the signaling domain comprises a co-stimulatory domain selected from the group consisting of CD28 and 4-1BB. Preferably, the signaling domain consists of a costimulatory domain selected from the group consisting of CD28 and 4-1BB. In another embodiment, the signaling domain further comprises a CD3ζ intracellular region. In this embodiment, the CAR may further comprise a signal peptide from B2M, CD8α, IgG1 or GM-CSFRα.

The present disclosure further provides a nucleic acid molecule encoding the NKG2A-targeting chimeric receptor as defined above, and a vector comprising the nucleic acid molecule.

As used herein, the term "vector" is an intermediary nucleic acid molecule used to transfer (exogenous) genetic material into a host cell, and in the host cell the nucleic acid molecule can be, for example, replicated and/or expressed. The vector generally includes targeting vectors and expression vectors. The "targeting vector" is a medium that delivers an isolated nucleic acid to the interior of a cell by, for example, homologous recombination or by using a hybridization recombinase specifically targeting a sequence at a site. The "expression vector" is a vector used for transcription of heterologous nucleic acid sequences (for example, those sequences encoding the chimeric receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, plasmid, virus (e.g., retrovirus, lentivirus, adenovirus, vaccinia virus, Rous sarcoma virus (RSV), polyoma virus, and adeno-associated virus (AAV), etc.), phage, phagemid, cosmid, and artificial chromosome (including BAC and YAC). The vector itself is usually a nucleic acid molecule, and usually is a DNA sequence containing an insert (transgene) and a larger sequence as "backbone" of the vector. Engineered vector typically also contains an origin of autonomous replication in the host cell (if stable expression of polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an in vitro transcription vector.

### Engineered immune cells

NKG2A binds to non-classical HLA-class I molecules, such as HLA-E, thereby inhibiting the activation of immune cells such as NK cells. Therefore, The introduction of exogenous NKG2A antibodies can inhibit the killing effect of NK cells by binding to NKG2A, which is especially useful in some cases (such as in the absence of HLA-class I molecules or in the preparation of universal CAR-T cells).

Therefore, in an aspect, the present disclosure further provides an engineered immune cell, which expresses the chimeric receptor comprising the anti-NKG2A antibody of the present disclosure, wherein the expression of at least one MHC-related gene is suppressed or silenced. In a preferred embodiment, the engineered immune cell also expresses a second chimeric receptor comprising a second NK inhibitory ligand, wherein the second NK inhibitory ligand is an antibody or fragment thereof targeting other NK inhibitory receptors, and/or natural ligands of other NK inhibitory receptors or NK inhibitory receptor binding regions comprised therein.

As used herein, MHC-related genes include MHC genes themselves (e.g., MHC-class I molecules and MHC-class II molecules), as well as genes that interact with MHC genes or regulate the expression of MHC genes. Examples of MHC class I molecules include, but are not limited to, HLA-A, HLA-B, HLA-C, B2M. Examples of MHC class II molecules include, but are not limited to, HLA-DPA1, HLA-DQA1, and HLA-DRA. Examples of genes that interact with MHC genes or regulate the expression of MHC genes include, but are not limited to, TAP1, TAP2, LMP2, LMP7, RFX5, RFXAP, RFXANK, and CIITA.

Accordingly, in an embodiment, inhibiting or silencing the expression of MHC-related genes refers to inhibiting or silencing the expression of one or more genes selected from: HLA-A, HLA-B, HLA-C, B2M, HLA-DPA, HLA-DQ, HLA-DRA, TAP1, TAP2, LMP2, LMP7, RFX5, RFXAP, RFXANK, CIITA and a combination thereof, and preferably selected from the group consisting of HLA-A, HLA-B, HLA-C, B2M, RFX5, RFXAP, RFXANK, CIITA and a combination thereof.

In an embodiment, the engineered immune cell expressing the chimeric receptor comprising the anti-NKG2A antibody of the present disclosure further comprises suppressed or silenced expression of at least one TCR/CD3 gene, examples of which include TRAC, TRBC, CD3γ, CD3δ, CD3ε, CD3ζ.

In a preferred embodiment, the engineered immune cells expressing the chimeric receptor of the present disclosure include suppressed or silenced expression of at least one TCR/CD3 gene and at least one MHC-related gene, wherein the at least one TCR/CD3 gene is selected from the group consisting of TRAC, TRBC, CD3γ, CD3δ, CD3ε, CD3ζ and a combination thereof; the at least one MHC-related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA and a combination thereof , and preferably selected from the group consisting of HLA-A, HLA-B, HLA-C, B2M, RFX5, RFXAP, RFXANK, CIITA and a combination thereof.

In a preferred embodiment, the at least one TCR/CD3 gene is selected from the group consisting of TRAC, TRBC and a combination thereof, and the at least one MHC-related gene is selected from the group consisting of B2M, RFX5, RFXAP, RFXANK, CIITA and a combination thereof. In an embodiment, the expression of TRAC or TRBC, and B2M of the engineered immune cells is inhibited or silenced. In an embodiment, the expression of TRAC or TRBC, and CIITA of the engineered immune cells is inhibited or silenced. In a preferred embodiment, the expression of TRAC or TRBC, B2M and CIITA of the engineered immune cells is suppressed or silenced. In a preferred embodiment, the expression of TRAC or TRBC, B2M and RFX5 of the engineered immune cells is inhibited or silenced.

Methods for inhibiting gene expression or gene silencing are well known to those skilled in the art, including but not limited to, for example, mediating DNA breakage by meganucleases, zinc finger nucleases, TALE nucleases, or Cas enzymes in the CRISPR system, or inactivating genes through technologies such as antisense oligonucleotides, RNAi, shRNA, etc.

In an embodiment, the engineered immune cell provided by the present disclosure further express a chimeric antigen receptor targeting a tumor antigen.

In an embodiment, the chimeric antigen receptor targets a tumor antigen selected from the group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAIX, LMP2, gplOO, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, Claudin18.2, NKG2D and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY- ESO-1, CD79a, CD79b, GPC3, Claudin18.2, NKG2D, and any combination thereof. Depending on the antigen to be targeted, the chimeric antigen receptor of the disclosure may be designed to include an antibody specific for the antigen. For example, if CD19 is the antigen to be targeted, an anti-CD19 antibody may be used in the chimeric antigen receptor of the disclosure.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell may be any T cell, such as in vitro cultured T cell, for example, primary T cell, or T cell from in vitro cultured T cell line, e.g., Jurkat, SupT1, etc., or T cell obtained from a subject. Examples of subject include humans, dogs, cats, mice, rats, and transgenic species thereof. The T cell may be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be at any stage of development including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell. In a preferred embodiment, the immune cell is a human T cell. The T cell may be isolated from the blood of a subject using a variety of techniques known to those of skill in the art, such as Ficoll.

The nucleic acid sequence encoding the chimeric receptor may be introduced into an immune cell using conventional methods known in the art (e.g., by transduction, transfection, transformation). "Transfection" is a process of introducing a nucleic acid molecule or polynucleotide (including a vector) into a target cell. An example is RNA transfection, i.e., the process of introducing RNA (e.g., in vitro transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation may be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation. After the nucleic acid or vector is introduced into the immune cells, those skilled in the art may amplify and activate the obtained immune cells by conventional techniques.

In an embodiment, the present disclosure further provides a plurality of engineered immune cells, wherein an immune cell expresses the chimeric receptor of the present disclosure and optionally a chimeric antigen receptor targeting a tumor antigen, and another immune cell expresses a second chimeric receptor targeting other NK inhibitory receptors. In this embodiment, the second chimeric receptor comprises a second NK inhibitory ligand, a transmembrane domain and a signaling domain, wherein the second NK inhibitory ligand, transmembrane domain and signaling domain are defined as described in the "Chimeric Receptors" section. In such embodiments, the plurality of engineered immune cells may be administered together or separately. In an embodiment, the plurality of immune cells may be in the same composition or in different compositions. Exemplary compositions of cells include those described in the following sections of this application.

### Antibody conjugate

In an aspect, the present disclosure provides an antibody conjugate comprising the anti-NKG2A antibody as defined herein and a second functional structure, wherein the second functional structure is selected from the group consisting of an Fc, a radioisotope, a structure moiety for extending half-life, a detectable marker and a drug.

In an embodiment, the present disclosure provides an antibody conjugate comprising the anti-NKG2A antibody as defined in the present disclosure and Fc. As used herein, the term "Fc" is used to define the C-terminal region of an immunoglobulin heavy chain, and includes natural Fc and variant Fc. "Natural Fc" refers to a molecule or sequence comprising a non-antigen-binding fragment, whether monomeric or multimeric, produced by digestion of an intact antibody. The source of immunoglobulin from which natural Fc is produced is preferably of human origin. Natural Fc fragments are composed of monomeric polypeptides that can be linked as dimers or multimers through covalent linkages (e.g., disulfide bonds) and non-covalent linkages. Depending on the class (e.g., IgG, IgA, IgE, IgD, IgM) or subtype (e.g., IgG1, IgG2, IgG3, IgA1, IgGA2), natural Fc molecules have 1-4 intermolecular disulfide bonds between monomeric subunits. An example of a natural Fc is a disulfide-linked dimer produced by papain digestion of IgG (see Ellison et al. (1982), Nucleic Acids Res. 10:4071-9). The term "natural Fc" as used herein generally refers to monomeric, dimeric and multimeric forms. A "variant Fc" refers to an amino acid sequence that differs from that of a "natural" or "wild-type" Fc by virtue of at least one "amino acid modification" as defined herein, also referred to as an "Fc variant". Thus, "Fc" also includes single-chain Fc (scFc), i.e., a single-chain Fc consisting of two Fc monomers linked by a polypeptide linker, which is capable of naturally folding into a functional dimeric Fc region. In an embodiment, the Fc is preferably the Fc of a human immunoglobulin, more preferably the Fc of a human IgG1.

In an embodiment, the present disclosure provides an antibody conjugate comprising the anti-NKG2A antibody as defined in the present disclosure and a radioactive isotope. Examples of radioisotopes useful in the present disclosure include, but are not limited to, At²¹¹, I1¹³¹, I¹²⁵, Y⁹⁰, Re¹⁸⁶, Re¹⁸⁸, Sm¹⁵³, Bi²¹², P³², Pb²¹², ^{99m}Tc, ¹²³I, ¹⁸F, and ⁶⁸Ga.

In an embodiment, the present disclosure provides an antibody conjugate comprising the anti-NKG2A antibody as defined in the present disclosure and a structure moiety for extending half-life selected from the group consisting of an albumin-binding structure, a transferrin-binding structure, a polyethylene glycol molecule, a recombinant polyethylene glycol molecule, a human serum albumin, a fragment of human serum albumin, and a polypeptide binding to human serum albumin (including antibody).

In an embodiment, the present disclosure provides an antibody conjugate comprising the anti-NKG2A antibody as defined in the present disclosure and a detectable marker. The term "detectable marker" means herein a compound that produces a detectable signal. For example, the detectable marker may be an MRI contrast agent, a scintigraphy contrast agent, an X-ray imaging contrast agent, an ultrasound contrast agent, an optical imaging contrast agent. Examples of detectable markers include fluorophores (e.g., fluorescein, Alexa, or cyanine), chemiluminescent compounds (e.g., luminol), bioluminescent compounds (e.g., luciferase or alkaline phosphatase), enzymes (e.g., horseradish peroxidase, glucose-6-phosphatase, β-galactosidase), antibiotics (e.g., kanamycin, ampicillin, chloramphenicol, tetracycline, etc.) resistance genes, and contrast agents (e.g., nanoparticles or gadolinium). Those skilled in the art can select an appropriate detectable marker according to the detection system used.

In an embodiment, the present disclosure provides an antibody conjugate comprising the anti-NKG2A antibody as defined in the present disclosure and a drug conjugated to the anti-NKG2A antibody, such as a cytotoxin or an immunomodulator (i.e., an antibody-drug conjugate). Usually, the drug is covalently linked to the antibody, usually by a linker. In an embodiment, the drug is a cytotoxin. In another embodiment, the drug is an immunomodulator. Examples of cytotoxins include, but are not limited to, methotrexate, aminopterin, 6-mercaptopurine, 6-thioguanine, cytarabine, 5-fluorouracil, dacarbazine, nitrogen mustard, thiotepa, chlorambucil, melphalan, carmustine (BSNU), lomustine (CCNU), 1-methylnitrosourea, cyclophosphamide, nitrogen mustard, busulfan, dibromomannitol, streptozocin, mitomycin, cisdichlorodiamine platinum (II) (DDP), cisplatin, carboplatin, zorubicin, doxorubicin, detorubicin, carminomicin, idarubicin, epirubicin, mitoxantrone, actinomycin D, bleomycin, calicheamicin, mithramycin, anthramycin (AMC), vincristine, vinblastine, paclitaxel, ricin, pseudomonas exotoxin, gemcitabine, cytochalasin B, gramicidin D, ethidium bromide, emetine, etoposide, teniposide, colchicine, mitoxantrone, 1-dehydrotestosterone, glucocorticoid, procaine, tetracaine, lidocaine, propranolol, puromycin, procarbazine, hydroxyurea, asparaginase, corticosteroids, mitotane (O,P'-(DDD)), interferon, and a combination thereof. Examples of immunomodulators include, but are not limited to, ganciclovir, etanercept, tacrolimus, sirolimus, voclosporin, cyclosporine, rapamycin, cyclophosphamide, azathioprine, mycophenolate mofetil, methotrexate, glucocorticoid and analogs thereof, cytokines, stem cell growth factors, lymphotoxins, tumor necrosis factor (TNF), hematopoietic factors, interleukins (e.g., IL-1, IL-2, IL-3, IL-6, IL-10, IL-12, IL-18 and IL-21), colony-stimulating factors (e.g., G-CSF and GM-CSF), interferons (e.g., interferon-α, interferon-beta and interferon-gamma), stem cell growth factor designated "S1 factor", erythropoietin and thrombopoietin, or a combination thereof.

### Kits and pharmaceutical compositions

In another aspect, the present disclosure further provides a detection kit comprising the antibody, the multispecific antibody, the chimeric receptor or the antibody conjugate of the present disclosure.

In another aspect, the present disclosure further provides a pharmaceutical composition comprising the antibody, the chimeric receptor, the multispecific antibody, or the antibody conjugate described in the present disclosure, and one or more pharmaceutically acceptable excipients.

As used herein, the term "pharmaceutically acceptable excipient" refers to a carrier and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, antiadherent, glidant, antioxidant, flavoring agent, colorant, sweetener, solvent, cosolvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods comprise topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also may be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition containing, for example, the immune cell as described herein is generally provided in a form of solution, and preferably contains a pharmaceutically acceptable buffer agent.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic/preventive/diagnostic use

In another aspect, the present disclosure further provides a method for treating and/or preventing and/or diagnosing diseases associated with NKG2A expression, comprising administering to a subject the antibody, the multispecific antibody, the antibody conjugate or the pharmaceutical composition as described above.

In an embodiment, diseases associated with NKG2A expression include cancer, infectious diseases, inflammatory diseases and autoimmune diseases. Examples of cancers that can be treated with the antibodies of the invention include, but are not limited to: solid cancers, including bladder cancer, breast cancer, colon cancer, kidney cancer, liver cancer, lung cancer, ovarian cancer, prostate cancer, pancreatic cancer, gastric cancer, cervical cancer, thyroid cancer and skin cancer, including squamous cell carcinoma; lymphoid hematopoietic neoplasms, including leukemia, acute lymphocytic leukemia, chronic lymphocytic leukemia, acute lymphoblastic leukemia, B-cell lymphoma, T-cell lymphoma, Hodgkin's lymphoma , non-Hodgkin lymphoma, hairy cell lymphoma, and Burkitt lymphoma, and multiple myeloma; myeloid hematopoietic neoplasms, including acute and chronic myeloid leukemia, promyelocytic leukemia, and myelodysplastic syndrome; tumors of mesenchymal origin, including fibrosarcoma and rhabdomyosarcoma; other tumors, including melanoma, seminoma, teratocarcinoma, neuroblastoma, and glioma; central and peripheral nervous system tumors, including astrocytoma, neuroblastoma, glioma, and schwannoma; tumors of mesenchymal origin, including fibrosarcoma, rhabdomyosarcoma, and osteosarcoma; and other neoplasms, including melanoma, xeroderma pigmentosa, keratoacanthoma, seminoma, follicular carcinoma of the thyroid, and teratocarcinoma. Examples of infectious diseases that can be treated with the antibodies of the invention include, but are not limited to, infections caused by viruses, bacteria, protozoa, or fungi, where viruses include, for example, hepatitis A virus, hepatitis B virus, hepatitis C virus, influenza virus, varicella virus, adenovirus, herpes simplex type 1 (HSV-1), herpes simplex type 2 (HSV-2), rinderpest, rhinovirus, echovirus, rotavirus, respiratory syncytial virus, papilloma virus, cytomegalovirus, arbovirus, coxsackie virus, mumps virus, measles virus, rubella virus, polio virus, human immunodeficiency virus type 1 or 2 (HIV-1 , HIV-2); bacteria include, for example, *Staphylococcus, Streptococcus, Bacillus, Lactobacillus, Listeria, Corynebacterium diphtheriae* and the like. Examples of inflammatory diseases that can be treated with the antibodies of the invention include, but are not limited to: adrenalitis, alveolitis, angiocholecystitis, appendicitis, balanitis, blepharitis, bronchitis, bursitis, carditis, cellulitis, cervicitis, cholecystitis, chorditis vocalis, cochleitis, colitis, conjunctivitis, cystitis, dermatitis, diverticulitis, encephalitis, endocarditis, esophagitis, eustachitis, fibrositis, folliculitis, gastritis, gastroenteritis, gingivitis, glossitis, hepatosplenitis, keratitis, otitis interna, laryngitis, lymphangitis, mastitis, otitis media, meningitis, metritis, mucositis, etc. Examples of autoimmune diseases that can be treated with the antibodies of the invention include, but are not limited to: hemolytic anemia, pernicious anemia, polyarteritis nodosa, systemic lupus erythematosus, Wegener's granulomatous disease, autoimmune hepatitis, Behcet's disease, Crohn's disease, primary biliary cirrhosis, scleroderma, ulcerative colitis, sjogren's syndrome, type 1 diabetes, uveitis, Graves' disease, Alzheimer's disease, psoriasis, vitiligo, etc.

the tumor antigens targeted by the chimeric antigen receptors. For example, when the chimeric receptor comprising an anti-NKG2A antibody of the present disclosure is co-expressed or co-administered with a CD19-targeting chimeric antigen receptor, the disease that can be treated is a disease associated with CD19 expression, such as a B-cell malignancy, including acute lymphocytic leukemia (B-ALL), chronic B-lymphocytic leukemia (B-CLL), B-cell Hodgkin's lymphoma (B-HL) and non-Hodgkin's lymphoma (B-NHL), etc. In this embodiment, the chimeric receptor comprising an anti-NKG2A antibody is used to inhibit the killing of reinfused engineered immune cells by NK cells, and the chimeric antigen receptor is used to direct the killing to target cells by binding to tumor antigens.

The present disclosure will be described in detail below with reference to the accompanying drawings and examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Examples

### Example 1. Preparation of NKG2A antibodies

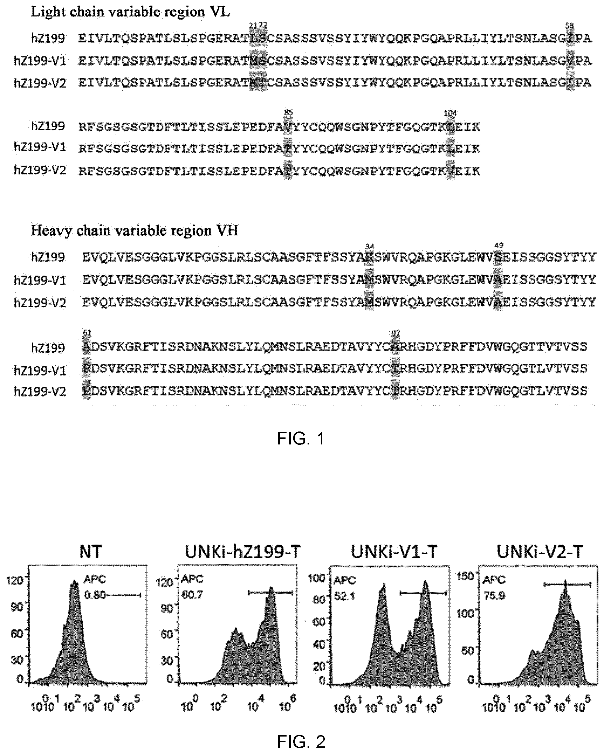

In order to enhance the affinity and specificity of the antibody, a new anti-NKG2A antibody was prepared based on the humanized Z199 antibody (hZ199) by back mutation. hZ199 comprises CDR-L1 as set forth in SEQ ID NO: 1, CDR-L2 as set forth in SEQ ID NO: 2, CDR-L3 as set forth in SEQ ID NO: 3, CDR-H1 as set forth in SEQ ID NO: 4, CDR-H2 as set forth in SEQ ID NO: 5, and CDR-H3 as set forth in SEQ ID NO: 6. The amino acid sequence of the light chain variable region set forth in SEQ ID NO: 7, the amino acid sequence of the heavy chain variable region set forth in SEQ ID NO: 8, and the full-length amino acid sequence set forth in SEQ ID NO: 9. Methods of back mutation are known in the art, for example, designing back mutation primers according to some amino acids (mainly the framework region) in the hZ199 sequence that may affect the affinity of the antibody, designing 5 mutation points in the light chain variable region, and 4 mutation points in the heavy chain variable region, and finally two back mutant antibodies were obtained by combination, named hZ199-V1 and hZ199-V2, whose sequences are shown in Table 1 and Figure 1.

**Table 1. Sequences of hZ199 and its back mutant antibodies**

| | hZ199 | hZ199-V1 | hZ199-V2 |
|---|---|---|---|
| VL | SEQ ID NO: 7 | SEQ ID NO: 10 | SEQ ID NO: 13 |
| VH | SEQ ID NO: 8 | SEQ ID NO: 11 | SEQ ID NO: 11 |
| full length | SEQ ID NO: 9 | SEQ ID NO: 12 | SEQ ID NO: 14 |

### Example 2. Preparation of UNKi-T cells expressing chimeric receptors containing anti-NKG2A antibodies and verification of their functions

Sequences encoding the following proteins were synthesized and cloned into the pLVX vector (Public Protein/Plasmid Library (PPL), Cat. No.: PPL00157-4a): B2M signal peptide (SEQ ID NO: 29), hZ199 (SEQ ID NO: 9) or hZ199-V1 (SEQ ID NO: 12), CD28 hinge region (SEQ ID NO: 35), CD8α transmembrane region (SEQ ID NO: 17), CD28 costimulatory domain (SEQ ID NO: 21), to obtain hZ199 plasmid and hZ199-V1 plasmid, and the correct insertion of the target sequence in the plasmid was confirmed by sequencing.

Sequences encoding the following proteins were synthesized and cloned into the pLVX vector (Public Protein/Plasmid Library (PPL), Cat. No.: PPL00157-4a): CD8α signal peptide (SEQ ID NO: 31), hZ199-V2 (SEQ ID NO: 14), IgG4 hinge region (SEQ ID NO: 37), CD28 transmembrane region (SEQ ID NO: 19), 4-1BB co-stimulatory domain (SEQ ID NO: 23), to obtain hZ199-V2 plasmid, and the correct insertion of the target sequence in the plasmid was confirmed by sequencing.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask containing 293T cells. Viruses were collected at 24 hours and 48 hours, pooled, and ultracentrifuged (25000g, 4°C, 2.5 hours) to obtain concentrated lentivirus.

T cells were activated with DynaBeads CD3/CD28 CTSTM (Gibco, Cat. No. 40203D), and were further cultured for 1 day at 37°C and 5% CO2. Then, the concentrated lentivirus was added, and after 3 days of continuous culture, T cells expressing the chimeric receptor containing NKG2A antibody were obtained.

Then the CRISPR/Cas9 system was used to knock out TCR/CD3 components (specifically the TRAC gene) and MHC-related genes ( specifically B2M and RFX5) in wild-type T cells (i.e. NT cells) and T cells expressing chimeric receptors containing anti-NKG2A antibodies, to obtain Mock T cells, UNKi-hZ99-T cells (containing hZ199 plasmid), UNKi-V1-T cells (containing hZ199-V1 plasmid) and UNKi-V2-T cells (containing hZ199-V2 plasmid), respectively. Using FITC Mouse Anti-Human CD3 (BD Pharmingen, Cat. No. 555916) antibody, PE mouse anti-human HLA-I (R&D Cat. No. FAB7098P) and APC anti-human DR, DP, DQ (biolegend, Cat. No. 361714) antibody, the expression efficiency of CD3/HLA-I/HLA-II in the UNKi-T cells, Mock T cells and NT cells was detected by flow cytometry, and the results are shown in Table 2 below.

**Table 2. Gene expression efficiency in UNKi-T cells**

| Cell name | TCR/CD3 | B2M/HLA-I | RFX5/HLA-II |
|---|---|---|---|
| UNKi-hZ199-T | 2.1% | 13% | 12.8% |
| UNKi-V1-T | 4.5% | 17.9% | 11.3% |
| UNKi-V2-T | 3.9% | 16.8% | 11.5% |
| Mock T | 3.7% | 14.8% | 11.8% |
| NT | 98% | 98% | 87% |

It can be seen from Table 2 that the expression of CD3/HLA-I/HLA-II in UNKi-T cells and Mock T cells prepared in the present disclosure is effectively suppressed or silenced.

The expression level of anti-NKG2A scFv in the UNKi-T cells and Mock T cells was detected using Biotin-SP (long spacer) AffiniPure Goat Anti-Human IgG, F(ab') fragment specific antibody (Jackson ImmunoResearch, Cat. No. 109-065-097) and APC Streptavidin (BD, Cat. No. 554067) (Figure 2).

It can be seen that all the scFvs in the UNKi-T cells prepared in the present disclosure are effectively expressed.

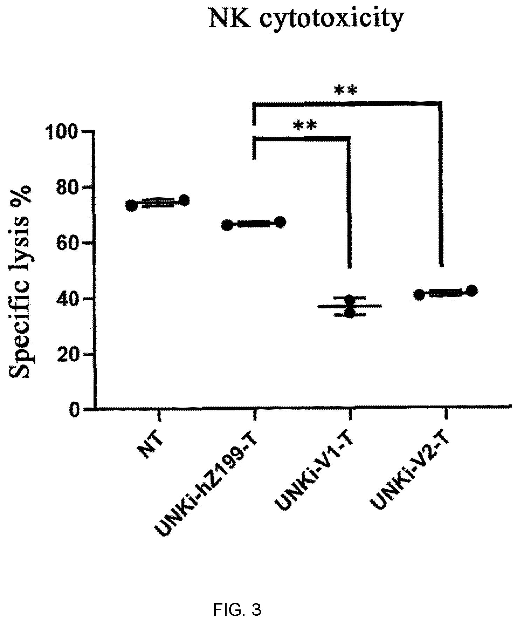

The inhibitory effect of the UNKi-T cells prepared by the present disclosure on the killing effect of NK cells was detected according to the following method: the UNKi-T cells and Mock-T cells prepared in the present disclosure were labeled with Far-Red (invitrogen, product number C34564). Then, the labeled UNKi-T cells and Mock T cells were plated into 96-well plates at a concentration of 1×10⁴ cells/well, and NK92 cells were added at an effector-to-target ratio of 2:1 for co-culture. After 16-18 hours, the proportion of T cells in the culture was detected by flow cytometry, and then the killing rate of NK cells on T cells was calculated. The results are shown in Figure 3 .

It can be seen from Figure 3 that, compared with NT cells not expressing chimeric receptors, the UNKi-T cells of the present disclosure significantly reduce the killing effect of NK cells on T cells. In addition, compared with the hZ199 antibody, the antibodies of the present disclosure (i.e., back mutant antibodies hZ199-V1 and hZ199-V2) have a better inhibitory effect on NK cell killing, indicating that the affinity of the antibodies of the present disclosure is greater than that of the hZ199 antibody.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. A NKG2A-targeting antibody, comprising a light chain variable region and a heavy chain variable region, **characterized in that** the light chain variable region comprises CDR-L1 as set forth in SEQ ID NO: 1, CDR-L2 as set forth in SEQ ID NO: 2, and CDR-L3 as set forth in SEQ ID NO: 3, and the heavy chain variable region comprises CDR-H1 as set forth in SEQ ID NO: 4, CDR-H2 as set forth in SEQ ID NO: 5, and CDR-H3 as set forth in SEQ ID NO: 6, wherein the light chain variable region comprises amino acid M at position 21, and amino acid T at position 85, and the heavy chain variable region comprises amino acid M at position 34, amino acid A at position 49, amino acid P at position 61, and amino acid T at position 97.

2. The antibody according to claim 1, **characterized in that** the light chain variable region comprises amino acid S or T at position 22, amino acid I or V at position 58, and amino acid L or V at position 104.

3. The antibody according to claim 1, **characterized in that** the light chain variable region has at least 90% identity to an amino acid sequence selected from the group consisting of SEQ ID NOs: 10 and 13, or has one or several conservative modifications of amino acids compared to SEQ ID NO: 10 or 13, the heavy chain variable region has at least 90% identity to the amino acid sequence as set forth in SEQ ID NO: 11 or has one or several conservative modifications of amino acids compared to SEQ ID NO: 11.

4. The antibody according to any one of claims 1-3, **characterized in that** the light chain variable region is selected from the group consisting of SEQ ID NOs: 10 and 13, and the heavy chain variable region is as set forth in SEQ ID NO: 11.

5. The antibody according to any one of claims 1-3, **characterized in that** the antibody has an amino acid sequence selected from the group consisting of SEQ ID NOs: 12 and 14.

6. A nucleic acid molecule encoding the antibody according to any one of claims 1-5.

7. A multispecific antibody comprising the antibody according to any one of claims 1-5 and one or more second antibodies or antigen-binding portions thereof that specifically bind to antigens different from NKG2A.

8. The multispecific antibody according to claim 7, **characterized in that** the one or more second antibodies or antigen binding portions thereof are selected from the group consisting of a full-length antibody, Fab, Fab', (Fab')₂, Fv, scFv, scFv-scFv, a minibody, a diabody or sdAb.

9. A vector comprising a nucleic acid molecule encoding the antibody according to any one of claims 1-5 or the multispecific antibody according to claim 7 or 8.

10. A host cell expressing the antibody according to any one of claims 1-5 or the multispecific antibody according to claim 7 or 8.

11. A chimeric receptor comprising one or more NK inhibitory ligands, a transmembrane domain and a signaling domain, wherein the one NK inhibitory ligand or at least one of the more NK inhibitory ligands comprise the antibody according to any one of claims 1-5 or the multispecific antibody according to claim 7 or 8, and the signaling domain comprises one or more co-stimulatory domains.

12. The chimeric receptor according to claim 11, **characterized in that** the chimeric receptor comprises two NK inhibitory ligands, wherein a first NK inhibitory ligand comprises the antibody according to any one of claims 1-5 or the multispecific antibody according to claim 7 or 8, wherein a second NK inhibitory ligand is selected from: (1) an antibody or a fragment thereof targeting a NK inhibitory receptor selected from the group consisting of: NKG2A, NKG2B, CD94, LlR1,LIR2, LIR3, KIR2DL1, KIR2DL2/3, KIR3DL1, CEACAM1, LAIR1, and KLRG1; and/or (2) HLA-E, HLA-F, HLA-G, cadherin, collagen, OCIL, sialic acid, PD-L1, PD-L2, CD155, CD112, CD113, Gal-9, FGL1, and NK inhibitory receptor binding regions comprised therein.

13. The chimeric receptor according to claim 11 or 12, **characterized in that** the signaling domain consists of one or more co-stimulatory domains.

14. The chimeric receptor according to claim 11 or 12, **characterized in that** the signaling domain further comprises a CD3ζ intracellular region.

15. The chimeric receptor according to any one of claims 11-14, **characterized in that** the co-stimulatory domain is selected from the intracellular region of CD28 or 4-1 BB.

16. An engineered immune cell expressing the chimeric receptor according to any one of claims 11-15, **characterized in that** expression of at least one MHC-related gene is suppressed or silenced.

17. The engineered immune cell according to claim 16, **characterized in that** the MHC-related gene is selected from the group consisting of: HLA-A, HLA-B, HLA-C, B2M, HLA-DPA, HLA-DQ, HLA-DRA, TAP1, TAP2, LMP2, LMP7, RFX5, RFXAP, RFXANK, CIITA, and a combination thereof.

18. The engineered immune cell according to claim 16 or 17, **characterized in that** the engineered immune cell further comprises suppressed or silenced expression of at least one TCR/CD3 gene selected from the group consisting of TRAC, TRBC, CD3γ, CD3δ, CD3ε, and CD3ζ.

19. The engineered immune cell according to any one of claims 16-18, **characterized in that** the engineered immune cell also expresses a chimeric antigen receptor targeting a tumor antigen.

20. The engineered immune cell according to any one of claims 16-18, **characterized in that** the engineered immune cell is selected from the group consisting of a T cell, a NK cell, a NKT cell, a macrophage, and a dendritic cell.

21. An antibody conjugate comprising the antibody according to any one of claims 1-5 or the multispecific antibody according to claim 7 or 8, and a second functional structure, wherein the second functional structure is selected from the group consisting of an Fc, a radioisotope, a structure moiety for extending half-life, a detectable marker and a drug.

22. A detection kit comprising the antibody according to any one of claims 1-5, the multispecific antibody according to claim 7 or 8, the chimeric receptor according to any one of claims 11-15, or the antibody conjugate according to claim 21.

23. A pharmaceutical composition comprising the antibody according to any one of claims 1-5, the multispecific antibody according to claim 7 or 8, the chimeric receptor according to any one of claims 11-15, the engineered immune cell according to any one of claims 16-20 or the antibody conjugate according to claim 21, and one or more pharmaceutically acceptable excipients.

24. Use of the antibody according to any one of claims 1-5, the multispecific antibody according to claim 7 or 8, the chimeric receptor according to any one of claims 11-15, the engineered immune cell according to any one of claims 16-20, the antibody conjugate according to claim 21 or the pharmaceutical composition according to claim 23 in the preparation of a medicine for treating and/or preventing and/or diagnosing a disease associated with NKG2A expression.
